# EUROPEAN PATENT APPLICATION

(11) **EP 4 635 341 A1**
(43) Date of publication of application: **22.10.2025**
(21) Application number: 22968557.3
(22) Date of filing: 16.12.2022
(51) Int. Cl.: A24F 40/57, A24F 40/53, A24F 40/85

(54) **INHALATION DEVICE, CONTROL METHOD, AND PROGRAM**

(71) Applicant: Japan Tobacco Inc., Tokyo 105-6927 (JP)
(72) Inventor: FUJIKI, Takashi, Tokyo 130-8603 (JP)
(74) Representative: Hoffmann Eitle
(86) International application number: PCT/JP2022/046466
(87) International publication number: WO 2024/127648

(57) **Abstract**

An MCU (1) of an inhalation device (100): operates a heating unit (121C) when a stick-type base material (150) is housed in a housing unit (140C); and operates the heating unit (121C) in response to removal of the stick-type base material (150) from the housing unit (140C) after operation of heating the stick-type base material (150) is finished.

## Description

### TECHNICAL FIELD

The present disclosure relates to an inhalation device, a control method, and a program for generating an aerosol from a base material having an aerosol source.

### BACKGROUND ART

Conventionally, for example, inhalation devices have been known to generate an aerosol with a flavor component and deliver the generated aerosol to a user in an inhalable manner. Such inhalation devices typically deliver the aerosol generated by heating a base material, which comprises an aerosol source, with a heating unit (also referred to as a "heating element"), which is an electrically resistive or inductively heated heater.

A housing unit into which the base material is inserted may become soiled as the inhalation device is used. For example, a portion of the aerosol source from the base material could spill into the housing unit, and adhere to the housing unit due to liquid (for example, water) in the housing unit. Also, a portion of the aerosol generated by heating the base material could become liquid and adhere to the housing unit. For cleaning the housing unit, for example, PTL 1 discloses cleaning the inside of a cavity by inserting a cleaning article (for example, a brush) or adding a cleaning component into the cavity.

### CITATION LIST

### PATENT LITERATURE

PTL 1 Published Japanese translation of PCT international publication for patent application 2012-513750

### SUMMARY OF INVENTION

### TECHNICAL PROBLEM

However, there is scope for improvement from the point of view of convenience of cleaning an inhalation device.

The present disclosure provides an inhalation device, control method, and program that improves the convenience of cleaning the inhalation device.

### SOLUTION TO PROBLEM

One aspect of the present disclosure is
an inhalation device for generating an aerosol from a base material that contains an aerosol source, the inhalation device comprising:
a housing unit that houses the base material;
a heating unit that heats the housing unit; and
a control unit that controls the heating unit,
wherein the control unit
   operates the heating unit when the base material is housed in the housing unit, and
   operates the heating unit in response to the base material being removed from the housing unit after completion of an operation of heating the base material.

Also, one aspect of the present disclosure is
a control method executed by a computer that controls operation of an inhalation device that generates an aerosol from a base material having an aerosol source, wherein
the inhalation device comprises:
   a housing unit that houses the base material;
   a heating unit that heats the housing unit, and
the computer
   operates the heating unit when the base material is housed in the housing unit, and
   operates the heating unit in response to the base material being removed from the housing unit after completion of an operation of heating the base material.

Also, one aspect of the present disclosure is
a program that causes a computer that controls operation of an inhalation device that generates aerosol from a base material having an aerosol source to perform a predetermined process, wherein
the inhalation device comprises:
   a housing unit that houses the base material;
   a heating unit that heats the housing unit, and
the computer is caused to perform the process of
   operating the heating unit when the base material is housed in the housing unit, and
   operating the heating unit in response to the base material being removed from the housing unit after completion of the operation of heating the base material.

### ADVANTAGEOUS EFFECTS OF INVENTION

According to the present disclosure, the convenience of cleaning an inhalation device can be improved.

### BRIEF DESCRIPTION OF DRAWINGS

FIG. 1 is a schematic diagram illustrating a first configuration example of an inhalation device (inhalation device 100A);
FIG. 2 is a schematic diagram illustrating a second configuration example of an inhalation device (inhalation device 100B);
FIG. 3 is an overall oblique view of an inhalation device 100 according to an embodiment of the present disclosure;
FIG. 4 is an oblique view viewed from the front right side of an internal unit 10;
FIG. 5 is an oblique view viewed from the front left side of the internal unit 10;
FIG. 6 is an exploded oblique view of the internal unit 10;
FIG. 7 is a cross-sectional oblique view of a heater assembly 30;
FIG. 8 is a cross-sectional view at A-A in FIG. 5, showing the structure around a sensor FPC 73, stick detection sensor 12, and stick guide 31 (housing unit 140C);
FIG. 9 is a schematic diagram showing the progress of light emitted from the stick detection sensor 12, for the states in which a stick-type base material 150 is housed and not housed;
FIG. 10 is a graph showing detection and non-detection of the stick-type base material 150 based on the brightness;
FIG. 11 is a graph showing a stick heating profile and a cleaning heating profile;
FIG. 12 is a flowchart showing an example of processes executed by an MCU 1;
FIG. 13 is a diagram explaining how the MCU 1 determines whether or not to operate a heating unit 121C based on the cleaning heating profile, based on the remaining capacity (SOC) of a power supply unit 111C;
FIG. 14 is a graph for explaining regions (first region to third region) of brightness of the reflected light detected by the stick detection sensor 12; and
FIG. 15 is a graph showing a stick heating profile (for the case of high temperature and the case of low temperature), and a cleaning heating profile.

### DESCRIPTION OF EMBODIMENTS

The following is an explanation of an embodiment of the inhalation device, control method, and program according to the present disclosure, with reference to the figures. First, two configuration examples (a first configuration example and a second configuration example) to which the configuration of the inhalation device according to the present disclosure can be applied will be described. It should be noted that, hereinafter, the same or similar elements may be given the same or similar reference signs, and the description thereof may be omitted or simplified as appropriate.

### 1. Configuration Example of Inhalation Device

An inhalation device is a device for generating a substance to be inhaled by a user. Hereinafter, the substance generated by the inhalation device will be described as being an aerosol. Alternatively, the substance generated by the inhalation device may be a gas.

### (1) First Configuration Example

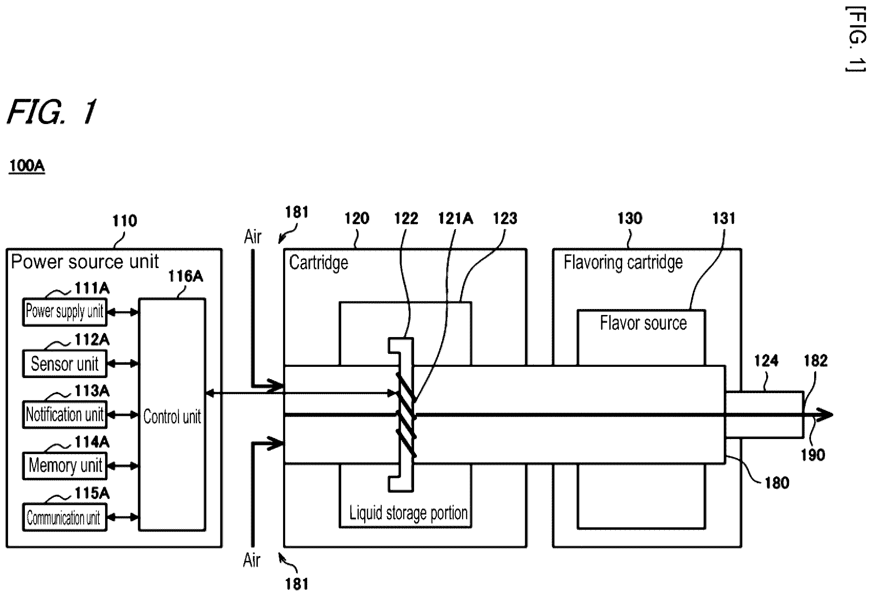

FIG. 1 is a schematic diagram illustrating a first configuration example of an inhalation device. As shown in FIG. 1, an inhalation device 100A of the present configuration example includes a power source unit 110, a cartridge 120, and a flavoring cartridge 130. The power source unit 110 comprises a power supply unit 111A, a sensor unit 112A, a notification unit 113A, a memory unit 114A, a communication unit 115A, and a control unit 116A. The cartridge 120 includes a heating unit 121A, a liquid guiding portion 122, and a liquid storage portion 123. The flavoring cartridge 130 includes a flavor source 131 and a mouthpiece 124. An air flow path 180 is formed in the cartridge 120 and the flavoring cartridge 130.

The power supply unit 111A stores electrical power. The power supply unit 111A then supplies the electrical power to each component of the inhalation device 100A in accordance with control performed by the control unit 116A. The power supply unit 111A may be configured, for example, by a rechargeable battery such as a lithium ion secondary battery.

The sensor unit 112A acquires various types of information relating to the inhalation device 100A. As an example, the sensor unit 112A is configured from a pressure sensor such as a condenser microphone, a flow rate sensor or a temperature sensor, and so on, and acquires values associated with inhalation by a user. As another example, the sensor unit 112A is configured by an input device, such as a button or switch, for accepting input of information from the user.

The notification unit 113A notifies the user of information. The information that the notification unit 113A notifies to the user is various including. for example, an SOC (State of charge) indicating a state of charge of the power supply unit 111A, pre-heating time at the time of inhalation, inhalation period, time that inhalation is possible, and so on. The notification unit 113A can be configured from, for example, a light-emitting device that emits light, a display device that displays images, a sound output device that outputs sound, a vibration device that vibrates, and so on.

The memory unit 114A stores various types of information for the operation of the inhalation device 100A. The memory unit 114A can be configured from a non-volatile storage medium such as a flash memory, for example.

The communication unit 115A is a communication interface capable of performing communication in accordance with any wired or wireless communication standard. Examples of communication standards that may be used include standards that employ Wi-Fi (registered trademark), Bluetooth (registered trademark), Bluetooth Low Energy (BLE) (registered trademark), Near-Field Communication (NFC), or Low Power Wide Area (LPWA), and so on.

The control unit 116A functions as an arithmetic processing device and a control device, and controls overall operation within the inhalation device 100A in accordance with various programs. The control unit 116A is realized by a central processing unit (CPU) or an electronic circuit such as a microprocessor, for example.

The liquid storage portion 123 stores an aerosol source. The aerosol source is atomized so as to generate an aerosol. The aerosol source is a polyhydric alcohol such as glycerol or propylene glycol, or a liquid such as water, for example. The aerosol source may include tobacco-derived or non-tobacco-derived flavor components. If the inhalation device 100A is a medical inhaler such as a nebulizer, the aerosol source may include a drug.

The liquid guiding portion 122 guides the aerosol source, which is the liquid stored in the liquid storage portion 123, from the liquid storage portion 123, and holds the aerosol source. The liquid guiding portion 122 is, for example, a wick formed by twisting a fibrous material such as glass fibers or a porous material such as a porous ceramic. In such a case, the aerosol source stored in the liquid store portion 123 is guided by the capillary effect of the wick.

The heating unit 121A heats the aerosol source to atomize the aerosol source, thereby generating the aerosol. In the example shown in FIG. 1, the heating unit 121A is configured as a coil wrapped around the liquid guiding portion 122. When the heating unit 121A generates heat, the aerosol source held in the liquid guiding portion 122 is then heated and atomized, generating the aerosol. The heating unit 121A generates heat when supplied with electricity from the power supply unit 111A. For example, electricity may be supplied to the heating unit 121A when the sensor unit 112A detects that the user has started inhaling and/or that predetermined information has been input. The supply of electricity to the heating unit 121A may then be stopped when the sensor unit 112A detects that the user has finished inhaling and/or that predetermined information has been input. Note that the inhalation action of the user on the inhalation device 100A is detectable, for example, based on pressure (internal pressure) exceeding a predetermined threshold in the inhalation device 100A. detected by a puff sensor.

The flavor source 131 is a component for imparting a flavor component to the aerosol. The flavor source 131 may include tobacco-derived or non-tobacco-derived flavor components.

The air flow path 180 is a flow path for air to be inhaled by the user. The air flow path 180 has a tubular structure with an air inflow hole 181, which is an inlet for air into the air flow path 180, and an air outflow hole 182, which is an outlet for air from the air flow path 180. Within the air flow path 180, the liquid guiding portion 122 is disposed upstream (closer to the air inflow hole 181), and the flavor source 131 is disposed downstream (closer to the air outflow hole 182). Air flowing in through the air inflow hole 181 upon inhalation by the user is mixed with the aerosol generated by the heating unit 121A and transported through the flavor source 131 to the air outflow hole 182, as shown by the arrow 190. When the mixed fluid of aerosol and air passes through the flavor source 131, the flavor component contained in the flavor source 131 is applied to the aerosol.

The mouthpiece 124 is a member that is held in the user's mouth during inhalation. The air outlet hole 182 is disposed in the mouthpiece 124. The user holds the mouthpiece 124 in their mouth to make it possible to draw the mixed fluid of aerosol and air into the oral cavity.

A configuration example of the inhalation device 100A has been described above. The inhalation device 100A is, of course, not limited to the configuration described above, and various configurations may be adopted, such as those illustrated below as examples.

As an example, the inhalation device 100A need not include the flavoring cartridge 130. In such a case, the cartridge 120 is provided with the mouthpiece 124.

As another example, the inhalation device 100A may include a plurality of types of aerosol sources. Other types of aerosol may be generated by a plurality of types of aerosol generated from the plurality of types of aerosol sources being mixed in the air flow path 180 to cause a chemical reaction.

Furthermore, the means for atomizing the aerosol source is not limited to heating provided by the heating unit 121A. For example, the means for atomizing the aerosol source may be vibration atomization or induction heating.

### (2) Second Configuration Example

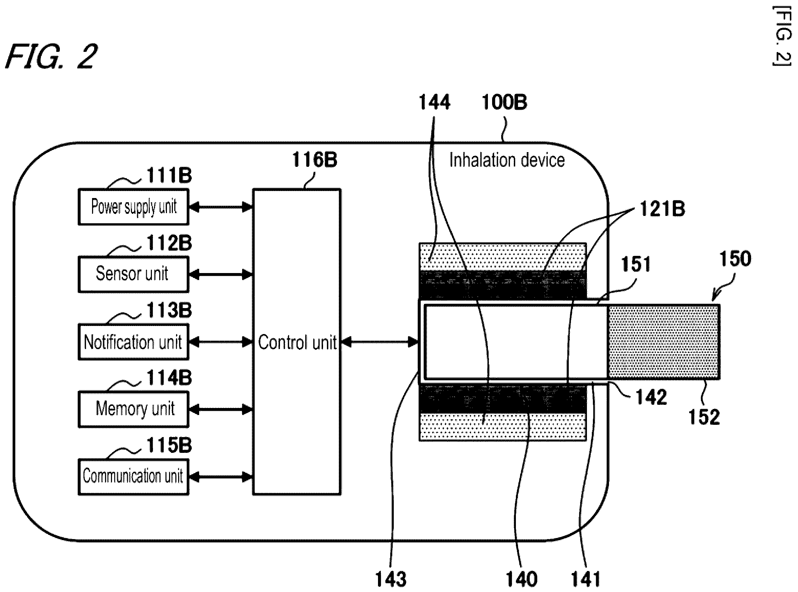

FIG. 2 is a schematic diagram illustrating a second configuration example of an inhalation device.

As illustrated in FIG. 2, an inhalation device 100B according to the present configuration example comprises a power supply unit 111B, a sensor unit 112B, a notification unit 113B, a memory unit 114B, a communication unit 115B, a control unit 116B, a heating unit 121B, a housing unit 140, and a heat insulating portion 144. While the inhalation device 100A of the first configuration example had the power source unit 110 containing the power supply unit 111A and the separate heating unit 121A, the inhalation device 100B of the second configuration example has an integral power supply unit 111B and heating unit 121B. That is, the inhalation device 100B of the second configuration example can also be described as having a power source unit with a built-in heating unit.

The power supply unit 111B, sensor unit 112B, notification unit 113B, memory unit 114B, communication unit 115B, and control unit 116B are each substantially identical to the corresponding component included in the inhalation device 100A according to the first configuration example.

The housing unit 140 has an internal space 141, and holds a stick-type base material 150 while accommodating a portion of the stick-type base material 150 in the internal space 141. The housing unit 140 has an opening 142 allowing the internal space 141 to communicate with the outside, and accommodates the stick-type base material 150 that has been inserted into the internal space 141 from the opening 142. For example, the housing unit 140 is a cylindrical body comprising the opening 142 and a bottom portion 143 serving as a bottom surface, and defines a columnar internal space 141. An air flow path for supplying air to the internal space 141 is connected to the housing unit 140. An air inflow hole, which is an inlet for air into the air flow path, is disposed in a side surface of the inhalation device 100, for example. An air outflow hole serving as an outlet for air from the air flow path to the internal space 141 is disposed in the bottom portion 143, for example.

The stick-type base material 150 comprises a base material portion 151 and a mouthpiece portion 152. The base material portion 151 includes an aerosol source. The aerosol source includes a tobacco-derived or non-tobacco-derived flavor component. If the inhalation device 100B is a medical inhaler such as a nebulizer, the aerosol source may include a drug. The aerosol source may be, for example, a liquid such as water and polyhydric alcohols such as glycerol and propylene glycol comprising the tobacco-derived or non-tobacco-derived flavor component, or else may be a solid comprising the tobacco-derived or non-tobacco-derived flavor component. In a state in which the stick-type base material 150 is being held in the housing unit 140, at least a portion of the base material portion 151 is accommodated in the internal space 141, and at least a portion of the mouthpiece portion 152 protrudes from the opening 142. Then, when the user holds the mouthpiece portion 152 protruding from the opening 142 in their mouth and inhales, air flows into the internal space 141 via the air flow path, which is not illustrated in the drawings, and reaches the inside of the user's mouth together with the aerosol generated from the base material portion 151.

In the example illustrated in FIG. 2, the heating unit 121B is configured in a film shape and is disposed so as to cover the outer periphery of the housing unit 140. Then, when the heating unit 121B generates heat, the base material portion 151 of the stick-type base material 150 is heated from the outer periphery, generating the aerosol.

The heat insulating portion 144 prevents heat transfer from the heating unit 121B to other components. For example, the heat insulating portion 144 is configured from a vacuum heat insulating material or an aerogel heat insulating material, or the like.

A configuration example of the inhalation device 100B has been described above. The inhalation device 100B is, of course, not limited to the configuration described above, and various configurations may be adopted, such as the examples illustrated below.

As one example, the heating unit 121B may have a blade-like form and may be arranged so as to protrude into the internal space 141 from the bottom portion 143 of the housing unit 140. In that case, the blade-like heating unit 121B is inserted into the base material portion 151 of the stick-type base material 150 and heats the base material portion 151 of the stick-type base material 150 from the inside. As another example, the heating unit 121B may be arranged so as to cover the bottom portion 143 of the housing unit 140. Furthermore, the heating unit 121B may be configured from a combination of two or more from among a first heating unit covering the outer circumference of the housing unit 140, a blade-like second heating unit, and a third heating unit covering the bottom portion 143 of the housing unit 140.

As another example, the housing unit 140 may comprise an opening/closing mechanism such as a hinge for opening/closing part of an external casing that forms the internal space 141. By opening/closing the casing, the housing unit 140 may then receive and grip the stick-type base material 150 that has been inserted into the internal space 141. In this case, the heating unit 121B may be provided on the clamping part of the housing unit 140, and may heat the stick-type base material 150 while pressing the same.

Furthermore, the means for atomizing the aerosol source is not limited to heating provided by the heating unit 121B. For example, the means for atomizing the aerosol source may be induction heating. In this case, the inhalation device 100B comprises at least an electromagnetic induction source such as a coil for generating a magnetic field, instead of the heating unit 121B. A susceptor which generates heat by means of induction heating may be provided in the inhalation device 100B, or may be contained in the stick-type base material 150.

The inhalation device 100B may further include the heating unit 121A, the liquid guiding portion 122, the liquid storage portion 123, and the air flow path 180 according to the first configuration example, and the air flow path 180 may supply air to the internal space 141. In this case, the mixed fluid of air and aerosol generated by the heating unit 121A flows into the internal space 141 and is further mixed with the aerosol generated by the heating unit 121B to reach the oral cavity of the user.

### 2. Configuration Examples of the Inhalation Device of the Present Disclosure

Next, an embodiment of the inhalation device (hereinafter referred to as the inhalation device 100) adopting the configuration of the inhalation device of the present disclosure is described in relation to the inhalation device 100B of the second configuration example previously described. Note that although the specific description is omitted, some of the configuration of the inhalation device 100 elaborated below can also be applied to the inhalation device 100A of the first configuration example.

### Overall Configuration of Inhalation Device

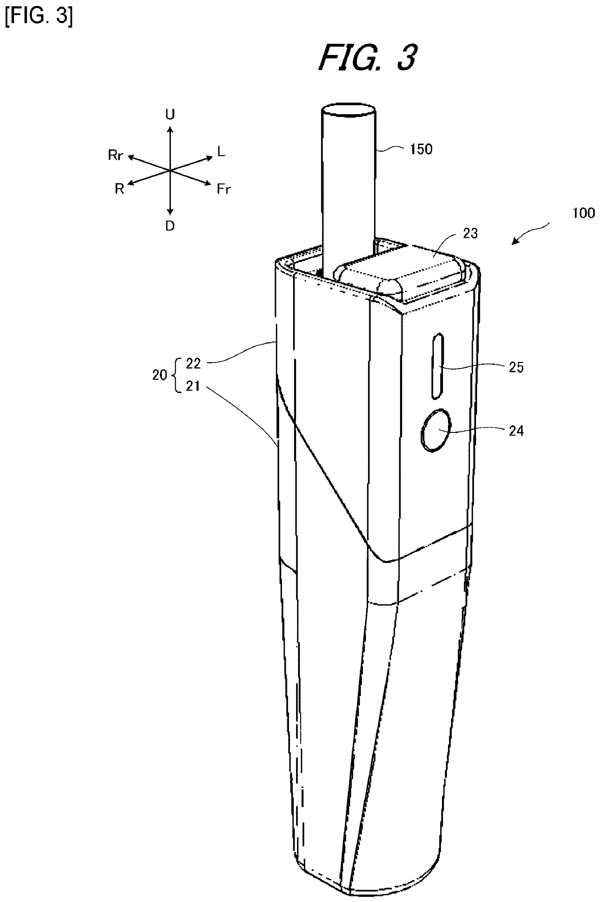

FIG. 3 is an overall oblique view of the inhalation device 100. In the following, in the inhalation device 100, the insertion and removal direction of the stick-type base material 150 relative to the inhalation device 100 is defined as the vertical direction, the sliding movement direction of a shutter 23 described below is defined as the front-rear direction, and the direction perpendicular to the vertical direction and the front-rear direction is defined as the left-right direction. Also, as shown in the figures, Fr is the front, Rr is the rear, L is the left side, R is the right side, U is up, and D is down.

The inhalation device 100 is preferably sized to fit in the hand, for example, having a rod shape. For example, the user holds the inhalation device 100 in one hand, with fingertips in contact with the front surface of the inhalation device 100. Note that the shape of the inhalation device 100 is not limited to a rod shape, but can be any shape (for example, rounded substantially cuboid shape or ovoid shape).

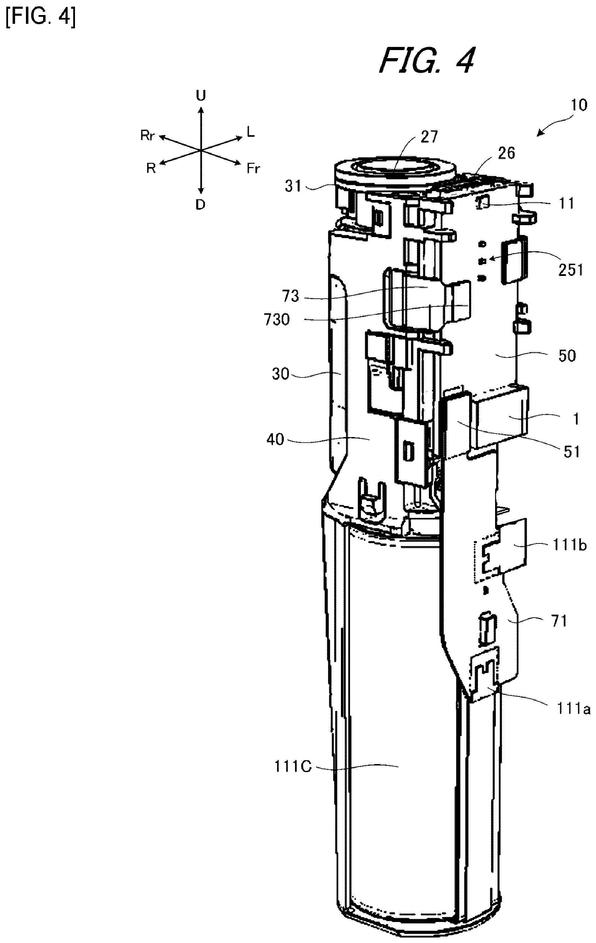

The inhalation device 100 comprises an internal unit 10 (see FIGS. 4 to 6), and a case 20 that constitutes the external appearance of the inhalation device 100. The case 20 has a lower case 21 and an upper case 22. A portion of the internal unit 10 is accommodated in the lower case 21, and the entire internal unit 10 is accommodated in the case 20 by covering the lower case 21 with the upper case 22 from above.

The upper surface of the inhalation device 100 is provided with an aperture 27 (see FIGS. 4 to 6) through which the stick-type base material 150 is inserted and removed, and a shutter 23 that is slidable in the front-rear direction. The aperture 27 is arranged at the rear of the upper surface of the inhalation device 100. The shutter 23 selectively takes an open state (front position) in which the aperture 27 is open to allow insertion and removal of the stick-type base material 150, and a closed state (rear position) in which the shutter 23 is positioned above the aperture 27 to obstruct the aperture 27. When inserting the stick-type base material 150 into the aperture 27, the user places the shutter 23 in the open state.

In the vicinity of the shutter 23, a shutter detection sensor 11 (see FIG. 4) is provided. The shutter detection sensor 11 detects whether or not the shutter 23 is in the open state. The shutter detection sensor 11 is an example of the sensor unit 112B of the inhalation device 100B of FIG. 2.

Also, a Universal Serial Bus (USB) port 26 (see FIG. 4) is provided on the upper surface of the inhalation device 100 arranged adjacent to the aperture 27. In the open state described above, the shutter 23 obstructs the USB port 26. On the other hand, in the closed state described above, the shutter 23 does not block the USB port 26 and the USB port 26 is open. The USB port 26 is configured to be electrically connectable with an external power source (not shown on the drawings) capable of supplying power to charge a power supply unit 111C (see FIG. 4). The USB port 26 is, for example, a receptacle in which a mating plug can be inserted. As an example, in the present embodiment, the USB port 26 is a USB Type-C receptacle.

An operation unit 24 and a light emitting unit 25 are provided on the front side of the inhalation device 100. The operation unit 24 is arranged below the light-emitting unit 25. In more detail, the operation unit 24 and the light-emitting unit 25 are one component of the internal unit 10 housed in the case 20, and are configured so that a part of the operation unit 24 and the light-emitting unit 25 are exposed through an opening formed in the front face of the case 20. The light emitting unit 25 is an example of the notification unit 113A of the inhalation device 100B of FIG. 2.

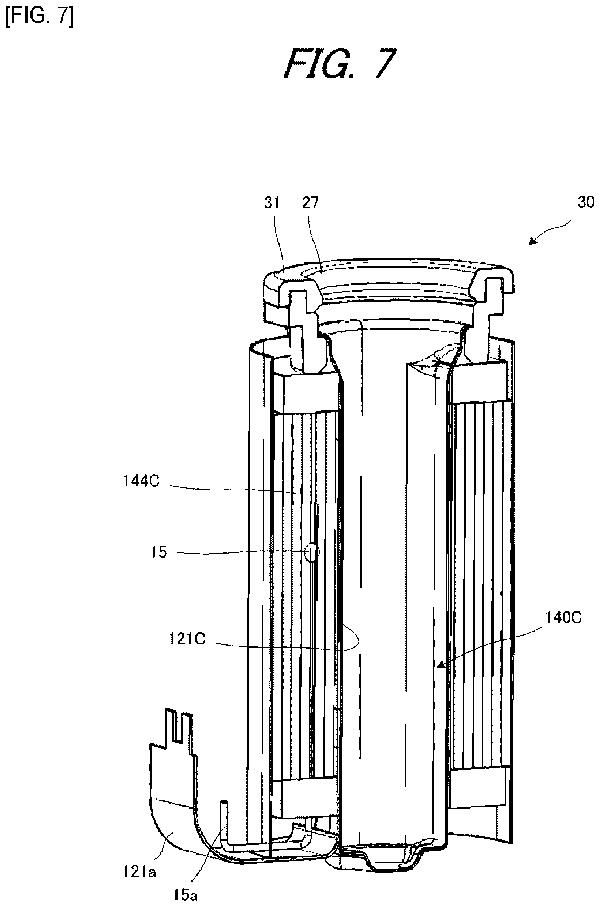

The operation unit 24 is a button-type switch that can be operated by a user, and is an input device for receiving input of information from a user. The operation unit 24 is connected to a main board 50 (see FIGS. 4 to 6) that is described later. The user depressing the operation unit 24, for example, activates a Micro Controller Unit (MCU) 1 (see FIGS. 4 to 6) or a heating unit 121C (see FIG. 7). Note that the MCU 1 acts as the control unit 116B in the inhalation device 100B. Also, the MCU 1 may be integrally provided with the functions of the communication unit 115B in addition to the functions of the control unit 116B in the inhalation device 100B. Furthermore, the MCU 1 may be configured from one IC, or may be configured from two or more ICs. For example, discharge control for the heating unit 121C and charging control to the power supply unit 111C may be performed in one IC or may be performed in separate ICs.

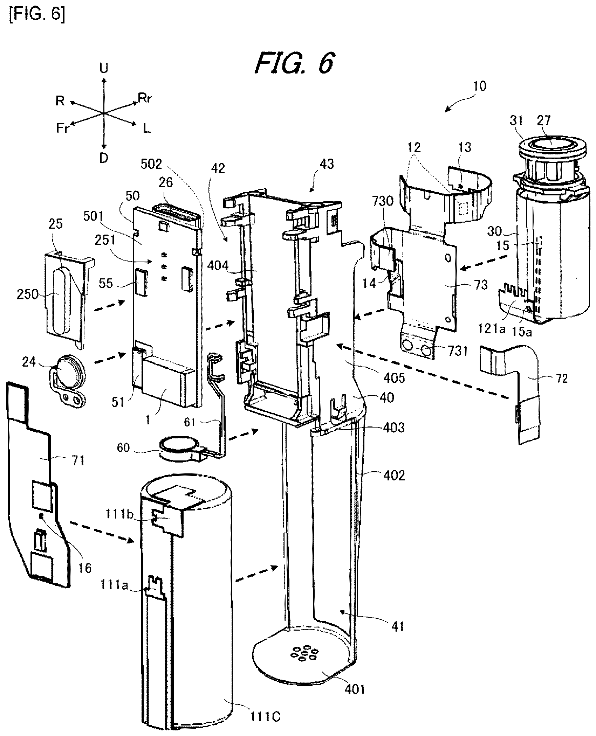

The light emitting unit 25 is configured from a light-emitting device such as, for example, a light-emitting diode (LED). In more detail, the light emitting unit 25 includes a plurality of LEDs 251 (see FIG. 6) provided on the main board 50, and a transparent cover 250 covering the plurality of LEDs 251 and allowing the transmission of light from the LEDs 251. A portion of the transparent cover 250 is exposed through an opening formed in the front face of the case 20. In the present embodiment, for example, it is assumed that the plurality of LEDs 251 is configured to be capable of emitting light in a plurality of colors, including blue, yellow, and red. Note that the number of light emitting elements can be set arbitrarily, for example there may be one light emitting element in the light emitting unit 25.

The light-emitting unit 25 emits light in a predetermined light-emitting mode by a command from the MCU 1 to notify the user of predetermined information. Here, the light emitting mode can be, for example, a light emitting color, but this is not a limitation, for example it can be the intensity of the illumination (in other words luminance) or the illumination pattern (for example, blinking at a predetermined time interval), and so on. Also, the predetermined information is, for example, operational information indicating whether the inhalation device 100 is powered on or not.

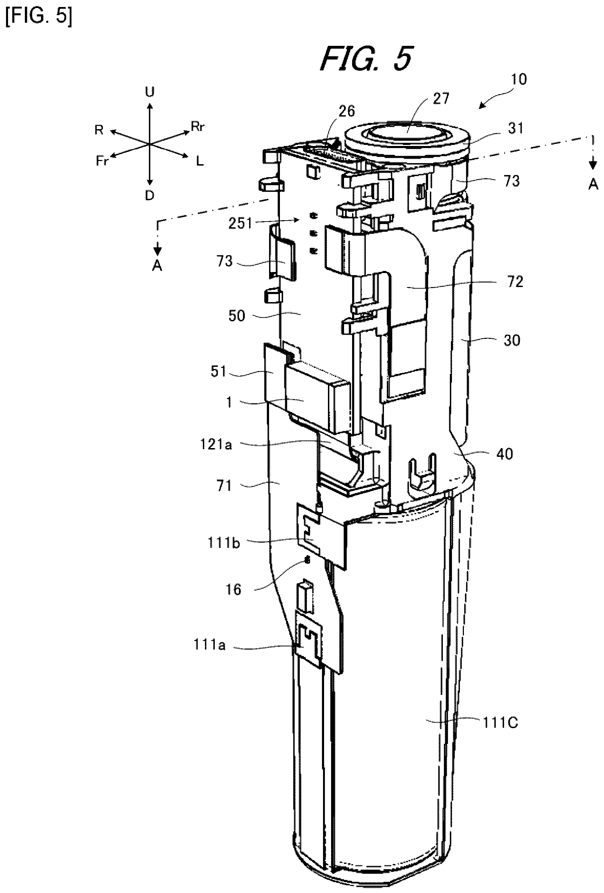

Next, the internal unit 10 of the inhalation device 100 of the present embodiment is described with reference to FIGS. 4 to 6. FIG. 4 is an oblique view of the internal unit 10 from the front right side, FIG. 5 is an oblique view of the internal unit 10 from the front left side, and FIG. 6 is an exploded oblique view of the internal unit 10. Note that the internal unit 10 is the inhalation device 100 from which the case 20 and the shutter 23 have been removed.

The internal unit 10 comprises a chassis 40, the main board 50, a vibration device 60, a heater assembly 30, the power supply unit 111C, a power supply board 71, a peripheral flexible printed circuit (FPC) 72, a sensor FPC 73, and various sensors. Note that the power supply board 71 may be a flexible circuit board, a rigid board as described below, or a combination of a flexible board and a rigid board, but here the example of a flexible circuit board is described as an example.

### Chassis

The chassis 40 comprises, as shown in the exploded oblique view of FIG. 6, a power supply retention portion 41 holding the power supply unit 111C, a board retention portion 42 holding the main board 50, and a heater retention portion 43 holding the heater assembly 30. The power supply retention portion 41 is located at the lower part of the chassis 40, and the board retention portion 42 and the heater retention portion 43 are located at the upper part of the chassis 40.

The power supply retention portion 41 has a cylindrical shape, with a portion of the side cut out, in other words a substantially semi-cylindrical shape. The power supply retention portion 41 has a bottom wall portion 401, a side wall portion 402 having a circular arc shape and extending upward from the bottom wall portion 401, and a top wall portion 403 provided at the upper end of the side wall portion 402. The power supply unit 111C is arranged in a space surrounded by the bottom wall portion 401, the side wall portion 402 and the top wall portion 403.

The board retention portion 42 is provided in a vertical wall portion 404 extending upward from the top wall portion 403 of the power supply retention portion 41. The board retention portion 42 is provided on one side (here on the front side) of the vertical wall portion 404 in the front-rear direction, and holds the main board 50.

The heater retention portion 43 is provided on the opposite side (here on the rear side) to the board retention portion 42 of the vertical wall portion 404 in the front-rear direction. The heater retention portion 43 has a space surrounded by the vertical wall portion 404, a left and right pair of wall portions 405 extending from the vertical wall portion 404 in a front-rear direction, and an upper surface of the top wall portion 403 of the power supply retention portion 41, and the heater assembly 30 is arranged in this space.

### Main Board

The main board 50 is a rigid board with a plurality of electronic components (elements) mounted on both sides. On the main board 50, the MCU 1, the LED 251, a charging IC (integrated circuit), a step-up DC/DC converter, and so on, are mounted. The main board 50 is held in the substrate retention portion 42 of the chassis 40 so that the element mounting surface is oriented in the front-rear direction. In FIG. 6, only a surface 501 of the main board 50 (here the front surface) is shown. Therefore, the charging IC and the step-up DC/DC converter mounted on the rear surface 502 (here on the back surface), are not shown.

In the lower region of the surface 501 of the main board 50, a power connection portion 51 is provided for electrical connection with the power supply unit 111C. The power connection portion 51 is electrically connected to the power supply unit 111C via the power supply board 71. The power source part 111C is a cylindrical lithium-ion secondary battery, and is an example of the power supply unit 111B of the inhalation device 100B of FIG. 2.

As shown in FIG. 6, the power supply unit 111C is provided with a positive electrode tab 111a and a negative electrode tab 111b. The power supply unit 111C is arranged in the power supply retention portion 41 of the chassis 40 so that the positive electrode tab 111a and the negative electrode tab 111b are arranged to the front. The power supply board 71 is arranged in front of the power supply unit 111C and the main board 50, and extends in the vertical direction. The power supply board 71 is connected to the positive and negative electrode tabs 111a, 111b of the power supply unit 111C and is connected to the power connection portion 51 of the main board 50. The power of the power supply unit 111C is transmitted to the main board 50 through an electrically conductive track formed in the power supply board 71, and is supplied to each electronic component. Also, the power supply board 71 is provided with a power supply temperature sensor 16. The power supply temperature sensor 16 is a sensor for measuring the temperature of the power supply unit 111C. The power supply temperature sensor 16 is, for example, a thermistor. The power supply temperature sensor 16 is an example of the sensor unit 112B of the inhalation device 100B of FIG. 2.

The USB port 26 is provided in an upper region of the rear surface 502 of the main board 50. The USB port 26 is electrically connected to the charging IC (not shown) by wires formed in the main board 50.

A heater connection is provided on the rear surface 502 of the main board 50, in addition to the charging IC and the step-up DC/DC converter, which are not shown. The charging IC performs charging control to supply (charge) the power input from the USB port 26 to the power supply unit 111C. The step-up DC/DC converter steps up the voltage of the power supplied from the power supply unit 111C, to supply the heating unit 121C (see FIG. 7).

A board connection part 121a extending from below the heater assembly 30 is connected to a heater connection part, to provide power to the heating unit 121C of the heater assembly 30. In this way the heating unit 121C of the heater assembly 30 is supplied with power from the power supply unit 111C via the main board 50.

### Vibration Device

The vibration device 60 is configured with a vibrating element such as, for example, a vibrating motor. As shown in FIG. 6, the vibration device 60 is arranged in the power supply retention portion 41 of the chassis 40 between the top surface of the power supply unit 111C and the top wall portion 403. Lead wires 61 of the vibration device 60 are connected to the peripheral FPC 72. The vibration device 60 vibrates in a predetermined vibration mode according to a command from the MCU 1, to notify the user of predetermined information. For example, at the start or end of heating of the stick-type base material 150, the vibration device 60 vibrates in a predetermined vibration mode to notify the user of the start or end of heating. The vibration device 60 is an example of the notification unit 113B of the inhalation device 100B of FIG. 2.

### Heater Assembly

FIG. 7 is a cross-sectional oblique view of the heater assembly 30.

The heater assembly 30 comprises the heating unit 121C, the housing unit 140C, and the insulating portion 144C. The heating unit 121C is, for example, a film heater, and is wound around the outer circumference of the housing unit 140C. Also, the heating unit 121C and the board connection part 121a may be configured with a single heater FPC.

Also, the heater assembly 30 is provided with a stick guide 31. The stick guide 31 is provided at the top of the heater assembly 30, and guides the insertion and removal of the stick-type base material 150 into the housing unit 140C. The stick guide 31 is a cylindrical-shaped member, has the aperture 27, and constitutes part of the housing unit 140C.

Also, the heater assembly 30 is provided with a heater temperature sensor 15 capable of measuring the temperature of the heating unit 121C. More specifically, the heater temperature sensor 15 is provided between the heating unit 121C and the heat insulating portion 144C, in contact with or close to the heating unit 121C. The heater temperature sensor 15 is, for example, a thermistor.

### Sensor FPC

As shown in FIG. 6, the sensor FPC 73 is arranged in the heater retention portion 43 between the vertical wall portion 404 and the heater assembly 30. The sensor FPC 73 is equipped with a stick detection sensor 12, a suction sensor 13, and a case temperature sensor 14. The stick detection sensor 12, the suction sensor 13, and the case temperature sensor 14 are examples of the sensor unit 112B of the inhalation device 100B of FIG. 2.

The stick detection sensor 12 is a sensor capable of detecting the stick-type base material 150 housed in the housing unit 140C. In the present embodiment, the stick detection sensor 12 is an optical sensor capable of detecting the stick-type base material 150 based on the amount of light reflected from the light emitted to the housing unit 140C. Here, amount of light is a concept that includes luminous flux, illuminance, luminous emittance, brightness, luminance, and so on. The optical sensor is for example an infrared ray (IR) sensor.

The suction sensor 13 is a sensor that detects a puff action (suction action) of a user. The suction sensor 13 comprises, for example, a capacitor microphone, a pressure sensor, or the like. The suction sensor 13 is provided in proximity to the stick guide 31 in the sensor FPC 73.

The case temperature sensor 14 is a sensor for measuring the temperature of the case 20. The case temperature sensor 14 is, for example, a thermistor. The case temperature sensor 14 is arranged in the sensor FPC 73 next to the inner surface of the case 20.

Also, the sensor FPC 73 is provided with a heater temperature sensor connection part 731 connected to the heater temperature sensor 15 of the heater assembly 30. The heater temperature sensor connection part 731 is provided in the lower part of the sensor FPC 73. In more detail, lead wires 15a are connected to the heater temperature sensor 15, and the heater temperature sensor connection part 731 is connected to the lead wires 15a extending from underneath the heater assembly 30.

The stick detection sensor 12, the suction sensor 13, the case temperature sensor 14, and the heater temperature sensor connection part 731 are connected to a board connection part 730 via an electrically conductive track formed in the sensor FPC 73. The board connection part 730 is connected to a sensor FPC connection part 55 provided in a central region of the surface 501 of the main board 50. In this way, the detection result of each sensor is output to the MCU 1, and so on, mounted on the main board 50.

In the inhalation device 100 configured in this way, when the open state of the shutter 23 is detected by the shutter detection sensor 11 and the stick-type base material 150 is detected by the stick detection sensor 12, the MCU 1 starts the heating by the heating unit 121C. When a user inhales on the mouthpiece portion 152 of the stick-type base material 150, aerosol is supplied into the user's mouth from the aerosol source of the stick-type base material 150 heated by the heating unit 121C. The suction sensor 13 detects the number of puffs, and the MCU 1 stops the heating after a predetermined number of puffs or after a predetermined time has elapsed. During heating of the inhalation device 100, the case temperature sensor 14, the heater temperature sensor 15, and the power supply temperature sensor 16 measure each temperature, and if it is determined that there is abnormal heating, the MCU 1 stops or reduces the heating by the heating unit 121C. The user can also operate the operation unit 24 to, for example, check the SOC of the power supply unit 111C, and so on. The light-emitting unit 25 (LED 251) and the vibration device 60 notify the user of various information such as the SOC of the power supply unit 111C, error indications, and so on. When the SOC of the power supply unit 111C drops, the user can connect an external power source to the USB port 26 to charge the power supply unit 111C.

### Stick Detection Sensor

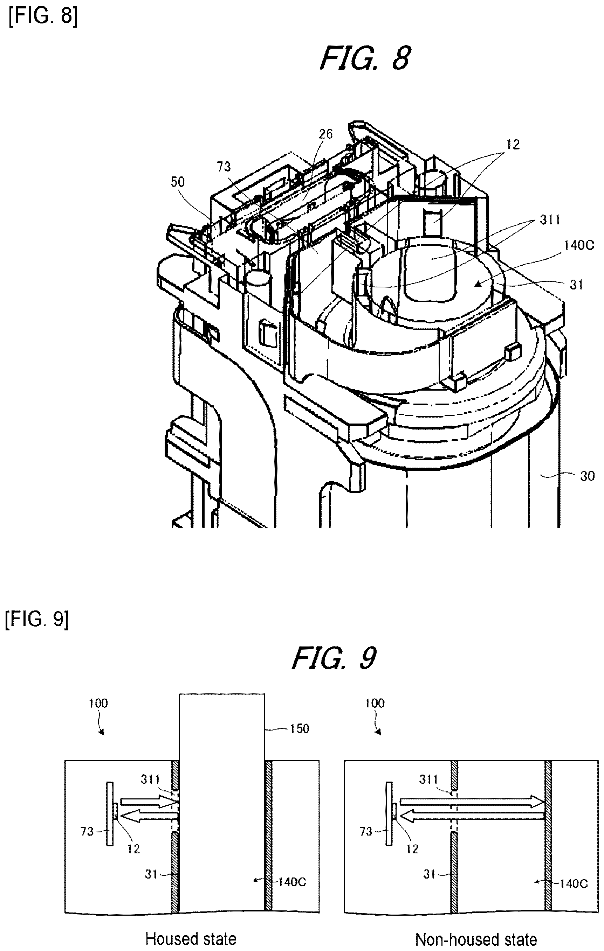

Next, details of the stick detection sensor 12 are described using FIGS. 8 and 9.

The stick detection sensor 12 is an optical sensor that illuminates light into the housing unit 140C and detects the amount of light reflected from the housing unit 140C. The MCU 1 is configured to be able to detect whether the stick-type base material 150 is accommodated in the housing unit 140C based on the amount of reflected light detected by the stick detection sensor 12. Here, the light emitted from and received by the stick detection sensor 12 is, for example, near infrared, in which case the stick detection sensor 12 is an IR sensor. In the following, the stick detection sensor 12 detects "brightness" as an example of the amount of light.

FIG. 8 is a cross-sectional view at A-A in FIG. 5, and shows the structure around the sensor FPC 73, the stick detection sensor 12, and the stick guide 31 (housing unit 140C). The sensor FPC 73 is a flexible member and is arranged around the housing unit 140C. The stick detection sensor 12 is provided on the sensor FPC 73. This allows the stick detection sensor 12 to be arranged around the housing unit 140C more easily than when the stick detection sensor 12 is provided on the rigid main board 50. Due to the greater degree of freedom in the arrangement, the inhalation device 100 can be made smaller.

The stick detection sensor 12 is arranged at a predetermined distance from the stick guide 31 to reduce the effect of heat from the stick guide 31 (housing unit 140C). Also, a transmission filter 311 that can transmit light is provided in part of the wall delimiting the housing unit 140C in the stick guide 31, and the sensor FPC 73 is arranged around the housing unit 140C so that the stick detection sensor 12 is opposite to and at a predetermined distance from the transmission filter 31. The portion of the stick guide 31 where the transmission filter 311 is not provided is configured to be non-transmissive to light.

As shown in FIG. 9, the stick detection sensor 12 emits light through the transmission filter 311 to the housing unit 140C and receives its reflected light. In the state in which the stick-type base material 150 is housed in the housing unit 140C (also referred to hereinafter as the housed state), light emitted from the stick detection sensor 12 is reflected on the surface of the stick-type base material 150 immediately after transmission through the transmission filter 311. The stick detection sensor 12 receives the reflected light reflected on the surface of the stick-type base material 150. On the other hand, in the state in which the stick-type base material 150 is not housed in the housing unit 140C (hereinafter also referred to as the unhoused state), the light emitted from the stick detection sensor 12 is transmitted through the transmission filter 311, passes through the housing unit 140C, and is reflected at the internal wall of the housing unit 140C. The stick detection sensor 12 receives light reflected at the inner wall of the housing unit 140C.

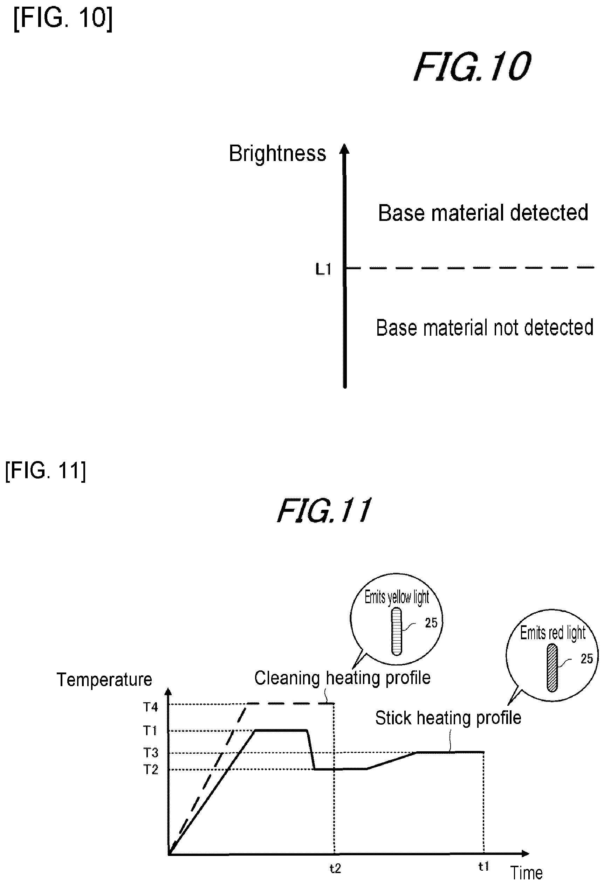

In this way, the distance that light travels from emission to receiving light in the housed state is shorter than in the unhoused state. Therefore, the brightness of the reflected light received by the stick detection sensor 12 in the housed state is higher than in the unhoused state. The MCU 1 performs detection of the stick-type base material 150 based on this difference in brightness between the housed state and the unhoused state. Specifically, as shown in FIG. 10, the MCU 1 detects the stick-type base material 150 when the brightness of the reflected light detected by the stick detection sensor 12 is greater than or equal to a predetermined value L1. On the other hand, the MCU 1 does not detect the stick-type base material 150 if the brightness of the reflected light detected by the stick detection sensor 12 is less than the predetermined value L1.

Note that in the present embodiment, two stick detection sensors 12 and two transmission filters 311 are provided. For example, the MCU 1 can be configured to not detect the stick-type base material 150 unless the detection result of both stick detection sensors 12 indicates the housed state of the stick-type base material 150.

### Example of Operation of Inhalation Device

Next, an example of operation of the inhalation device 100 will be described.

The inhalation device 100 is activated, for example, in response to the shutter 23 being in the open state. Specifically, the MCU 1 is activated in response to the shutter detection sensor 11 detecting the open state of the shutter 23. After activation of the MCU 1, operation of the heating unit 121C and so on is enabled. Here, the shutter detection sensor 11, for example, comprises a magnet provided in the shutter 23 and a Hall IC (integrated circuit) provided at the top end of the main board 50. Note that the MCU 1 may be activated in response to pressing the operation unit 24.

Next, an automatic heating mode and a manual heating mode will be explained as the modes for starting operation of the heating unit 121C.i

The automatic heating mode is a mode in which operation of the heating unit 121C is automatically started in response to a stick-type base material 150 being housed in the housing unit 140C. In the automatic heating mode, for example, the stick detection sensor 12 starts to emit and receive light and detect the amount of reflected light, in response to the shutter 23 being in the open state. When the automatic heating mode is selected, the MCU 1 starts the heating of the stick-type base material 150 after the stick-type base material 150 has been detected based on the detection result of the stick detection sensor 12.

The manual heating mode is a mode in which operation of the heating unit 121C is started in response to a request for heating from the user. When the manual heating mode is selected, the MUC 1 does not automatically start heating the stick-type base material 150 even though the stick-type base material 150 has been detected. The MCU 1 starts heating the stick-type base material 150 in response to a request from the user for heating. Here, the user requests heating by, for example, depressing the operation unit 24, or performs an inhaling operation on the inhalation device 100.

The user selects one mode from among the automatic heating mode and the manual heating mode. Mode selection is performed, for example, on the user's terminal (smartphone, or the like), and the MCU 1 receives the instruction information from the user's terminal via the communication unit 115A, and the mode selected by the user is set.

Next, heating of the stick-type base material 150 is described.

The MCU 1 operates the heating unit 121C based on a stick heating profile for heating the stick-type base material 150 when the stick-type base material 150 is housed in the housing unit 140C. The stick heating profile is information defining a time series transition of the target temperature, which is the target value of the temperature of the heating unit 121C, and is information for heating the stick-type base material 150. The stick heating profile is pre-stored in ROM, for example. The MCU 1 generates an aerosol from the stick-type base material 150 by controlling the temperature of the heating unit 121C based on the stick heating profile.

The solid line in FIG. 11 shows an example of a stick heating profile. According to the stick heating profile, the heating unit 121C can be heated to a maximum temperature T1 in conjunction with the start of heating control, and then temporarily lowered in temperature to T2, after which the temperature is raised again to T3. The heating control can then be ended when the elapsed time since the start of heating control is t1. In FIG. 11, when the temperature of the heating unit 121C reaches T1 and it is envisaged that the heating unit 121C is at a sufficiently high temperature, it is envisaged that a sufficient amount of aerosol has been generated, and the user can inhale. Note that the heating time before inhalation is possible is referred to as the pre-heating time.

Elaborating on the temperature control of the heating unit 121C that is based on the stick heating profile, the MCU 1 controls the temperature of the heating unit 121C based on the divergence between a target temperature corresponding to an elapsed time from the start of heating control, and the actual temperature of the heating unit 121C (also referred to as "actual temperature" hereinafter). More specifically, at this time, the MCU 1 controls the temperature of the heating unit 121C so that the time series transition of the actual temperature of the heating unit 121C is similar to the time series transition of the target temperature defined in the stick heating profile. Note that heating control of the housing unit 140C is similarly performed based on a cleaning heating profile that will be described later.

The stick heating profile is typically designed so that, when the user inhales the aerosol generated from the stick-type base material 150, the flavour tasted by the user is optimized. Therefore, controlling the temperature of the heating unit 121C based on the stick heating profile can optimize the flavor tasted by the user, and provide a high-quality smoking experience to the user.

As the inhalation device 100 is used, dirt adheres to the housing unit 140C. For example, a portion of the aerosol source of the stick-type base material 150 (for example, tobacco leaf) could spill out into the housing unit 140C, and may adhere to the housing unit 140C due to liquid (for example, water) within the housing unit 140C. Also, a portion of the aerosol generated by heating the stick-type base material 150 becomes liquid and may adhere to the housing unit 140C. Normally, if dirt is adhering to the housing unit 140C, the quality of the flavor tasted by the user declines, so it is desirable that the user periodically cleans the housing unit 140C.

Cleaning the housing unit 140C is performed, for example, by inserting a cleaning implement (for example, a cotton swab) with a cleaning agent (for example, a liquid substance such as alcohol or water) into the housing unit 140. In this way, the dirt adhering to the housing unit 140 can be removed. However, if the frequency of cleaning using the cleaning implement and cleaning agent is low, the dirt accumulates in the housing unit 140, and it becomes difficult to remove the dirt. On the other hand, if the frequency of cleaning using the cleaning implement and cleaning agent is high, it is tedious for the user.

Therefore, in the present embodiment, in response to the stick-type base material 150 being removed from the housing unit 140C after completion of the operation of the heating unit 121C based on the stick heating profile, the MCU 1 operates the heating unit 121C based on the cleaning heating profile for heating the housing unit 140C in which the stick-type base material 150 is not housed. Specifically, when the brightness of the reflected light becomes less than a predetermined value L1 after completion of the operation of the heating unit 121C based on the stick heating profile, the MCU 1 determines that the stick-type base material 150 has been removed from the housing unit 140C. In response to this determination result, the MCU 1 operates the heating unit 121C based on the cleaning heating profile. The cleaning heating profile is information that defines a time series transition of a target temperature, which is the target value of the temperature of the heating unit 121C, and is information for cleaning the inside of the housing unit 140C. The cleaning heating profile is pre-stored in ROM, for example. The cleaning heating profile is a heating profile that is different from the stick heating profile, and has different information such as target temperature and operating time, as described below.

In this way, the housing unit 140C is heated after the stick-type base material 150 is removed from the housing unit 140C, so the liquid (for example, water) present in the housing unit 140C evaporates. As a result, the portion of the aerosol source that was adhering to the housing unit 140C due to the liquid present in the housing unit 140C no longer adheres to the housing unit 140C. Therefore, the user can easily remove the portion of the aerosol source from the housing unit 140C by, for example, pointing the aperture 27 downward. Also, a portion of the aerosol that has become liquid and adheres to the housing unit 140C is evaporated by heating and removed from the housing unit 140C. The heating control for removing such dirt is performed immediately after removing the stick-type base material 150 (in other words, immediately after smoking), so dirt does not accumulate in the housing unit 140C, and the frequency of cleaning using cleaning tools and cleaning agent can be reduced. Therefore, the convenience of cleaning the inhalation device 100 can be improved.

Note that the timing for operating the heating unit 121C in response to removal of the stick-type base material 150 from the housing unit 140C includes the time at which the MCU 1 detects the stick-type base material 150 based on the detection result of the stick detection sensor 12, and the time at which a predetermined amount of time (a relatively short amount of time) has elapsed since the time at which the stick-type base material 150 was detected.

Here, the cleaning heating profile (dashed line in FIG. 11) is described in comparison with the stick heating profile, referring to FIG. 11.

The temperature of the heating unit 121C is raised to a maximum temperature T4 when heating control is started, in accordance with the cleaning heating profile, and is then maintained at this temperature T4. Then, when the elapsed time since the start of heating control is t2, the heating control is terminated.

The stick heating profile and the cleaning heating profile include operation time information for operating the heating unit 121C, and the operation time t2 of the cleaning heating profile is set shorter than the operation time t1 of the stick heating profile. The operation time t2 of the cleaning heating profile should be long enough to evaporate the moisture in the housing unit 140C. The operation time t2 of the cleaning heating profile is short, so excessive heating of the housing unit 140C in which no stick-type base material 150 is housed is reduced. Also, it is possible to reduce power consumption. Note that the MCU 1 may terminate the heating control based on the cleaning heating profile when the shutter 23 is closed before the operation time t2 has elapsed, or it may continue the heating control until the operation time t2 has elapsed even if the shutter 23 is closed before the operation time t2 has elapsed.

Also, the target temperature of the heating unit 121C in the cleaning heating profile is set higher than the target temperature of the heating unit 121C in the stick heating profile. Specifically, the maximum target temperature T1 of the stick heating profile is set to about 300°C, and the maximum target temperature T4 of the cleaning heating profile is set to a temperature higher than 300°C. By setting the target temperature of the cleaning heating profile high, the moisture in the housing unit 140C can be evaporated in a shorter time.

In the present embodiment, after a predetermined period of time has elapsed since the previous operation of the heating unit 121C which was performed in response to the stick-type base material 150 being removed from the housing unit 140C, the MCU 1 operates the heating unit 121C in response to the stick-type base material 150 being removed from the housing unit 140C after the operation of heating the stick-type base material 150 has been completed. Here, the predetermined period of time may be, for example, the period until the operation of heating the stick-type base material 150 has been performed a predetermined number of times (for example, 20 times), or a period (for example, 3 days) that is preset in the MCU 1 by the manufacturer of the inhalation device 100 or the like. For example, the predetermined period of time is counted or timed by the MCU 1.

To explain one example, after, for example, 20 stick-type base materials 150 have been heated since the end of the previous operation of the heating unit 121C based on the cleaning heating profile, the MCU 1 operates the heating unit 121C based on the cleaning heating profile in response to the 20th stick-type base material 150 being removed from the housing unit 140C. To explain another example, after, for example, three days have passed since the end of the previous operation of the heating unit 121C based on the cleaning heating profile, the MCU 1 operates the heating unit 121C based on the cleaning heating profile in response to the stick-type base material 150 being removed from the housing unit 140C after the operation of heating the stick-type base material 150 has ended. In this way, heating control is not performed continuously compared with when heating control based on the cleaning heating profile is performed each time one stick-type base material 150 is used, so power consumption can be reduced.

Also, in the present embodiment, the MCU 1 can selectively switch between a first mode in which the heating unit 121C is operated and a second mode in which the heating unit 121C is not operated in response to the stick-type base material 150 being removed from the housing unit 140C. In detail, the first mode is a mode in which the heating unit 121C is operated based on the cleaning heating profile in response to the stick-type base material 150 being removed from the housing unit 140C after operation of the heating unit 121C based on the stick heating profile has ended, as described above. The second mode is a mode in which the heating unit 121C is not operated when the stick-type base material 150 is removed from the housing unit 140C after the operation of the heating unit 121C based on the stick heating profile has ended.

For example, the first mode corresponds to an automatic heating mode, and the second mode corresponds to a manual heating mode. However, this correspondence relationship is not a limitation, and even when the automatic heating mode is selected, a separate mode may be provided in which the heating unit 121C is not operated when the stick-type base material 150 is removed from the housing unit 140C. Also, even when the manual heating mode is selected, a separate mode may be provided in which the heating unit 121C is automatically operated based on a cleaning heating profile in response to the stick-type base material 150 being removed from the housing unit 140C.

The MCU 1 selectively takes the first mode or the second mode, so the intention of the user who does not want heating control for cleaning after removing the stick-type base material 150 can be reflected.

### Example of Notification by the Notification Unit

Next, an example of notification to the user during heating will be described. Here, light emitted by the light emitting unit 25 (LED 251), which is an example of the notification unit 113B of FIG. 2, will now be described.

The light-emitting unit 25 notifies the user that the heating unit 121C is in operation. Specifically, it emits light in a predetermined light emitting mode when the heating unit 121C is operated based on the stick heating profile with the stick-type base material 150 housed in the housing unit 140C, and when the heating unit 121C is operated based on the cleaning heating profile after the stick-type base material 150 is removed from the housing unit 140C. Such notifications allow the user to easily and visually understand that the heating unit 121C is in operation. In particular, when the heating unit 121C is operating while the stick-type base material 150 is not housed, the user can see the light emitted by the light emitting unit 25 and take care not to bring their fingers close to the aperture 27, for example.

The light emitting unit 25 may emit light so that the light emission pattern when the heating unit 121C is operating based on the stick heating profile while the stick-type base material 150 is housed in the housing unit 140C is different from the light emission pattern when the heating unit 121C is operating based on the cleaning heating profile after the stick-type base material 150 has been removed from the housing unit 140C. For example, as shown in FIG. 11, the light emission color of the LED 251 is changed between the light emission pattern during heating control based on the stick heating profile and the light emission pattern during heating control based on the cleaning heating profile. Also, the light emission patterns may be distinguished by changing the number of LEDs 251 that emit light among the plurality of LEDs 251. By setting the light emission pattern to be different between the housed state and the unhoused state, the user can visually and easily understand that when the heating unit 121C is operating in the unhoused state, heating control is being performed differently from when the heating unit 121C is operating in the housed state.

Note that the notification by the notification unit 113B is not limited to light emission by the light emitting unit 25, but may be vibration by the vibration device 60, for example. Specifically, the vibration device 60 may vibrate during operation of the heating unit 121C to notify the user that the heating unit 121C is in operation. Also, the vibration device 60 may vibrate with a vibration pattern when the heating unit 121C is operating in the housed state that is different from the vibration pattern when the heating unit 121C is operating in the unhoused state.

### Example of Processes Performed by the Control Unit

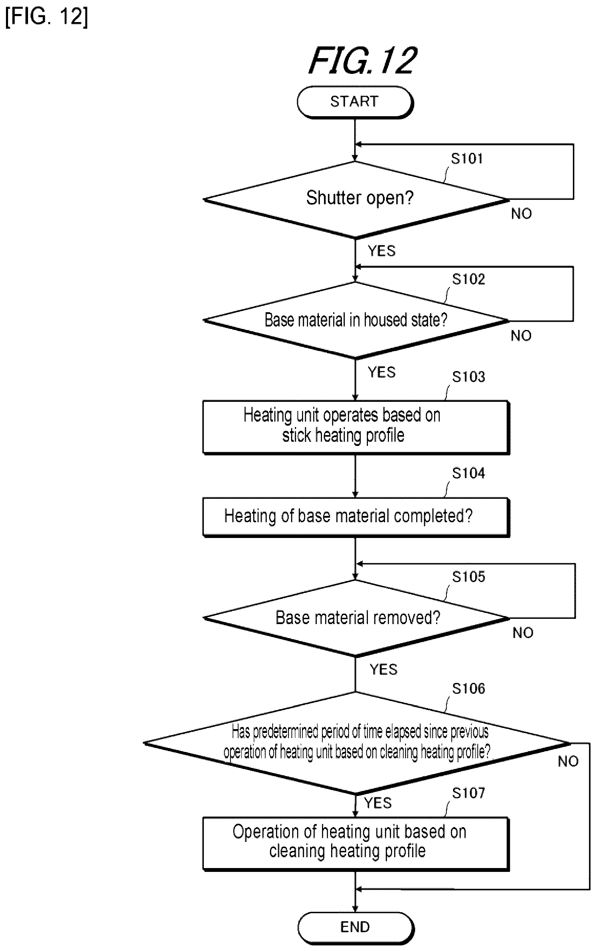

Next, examples of the processes performed by the MCU 1 are explained using the flowchart shown in FIG. 12.

The MCU 1 first determines whether the shutter 23 is in the open state (step S101). If the shutter 23 is not in the open state (step S101: NO), the MCU 1 repeatedly monitors step S101 until the shutter 23 is in the open state.

When the shutter 23 is in the open state (step S101: YES), the MCU 1 determines whether the stick-type base material 150 is housed in the housing unit 140 (step S102). Specifically, when the shutter 23 is in the open state, the stick detection sensor 12 starts operating, the MCU 1 acquires the detection result from the stick detection sensor 12, and determines whether or not the stick-type base material 150 is housed in the housing unit 140C. If the stick-type base material 150 is not housed in the housing unit 140C (step S102: NO), the MCU 1 repeatedly monitors step S102 until the stick-type base material 150 is housed in the housing unit 140C.

When the stick-type base material 150 is housed in the housing unit 140C (step S102: YES), the MCU 1 operates the heating unit 121C based on the stick heating profile (step S103). In this way, heating of the stick-type base material 150 is initiated, and aerosol is generated. Heating of the stick-type base material 150 ends when the operation time in the stick heating profile has elapsed, or when a predetermined number of inhalations has been performed since the start of heating of the stick-type base material 150 (step S104).

Next, the MCU 1 determines whether or not the stick-type base material 150 has been removed from the housing unit 140C after the operation of the heating unit 121C based on the stick heating profile has ended (step S105). If the stick-type base material 150 has not been removed from the housing unit 140C (step S105: NO), the MCU 1 repeatedly monitors step S105 until the stick-type base material 150 is removed from the housing unit 140C.

If the stick-type base material 150 has been removed from the housing unit 140C (step S105: YES), the MCU 1 determines whether or not a predetermined period of time has elapsed since the previous operation of the heating unit 121C based on the cleaning heating profile (step S106).

If the predetermined period of time has passed since the previous operation of the heating unit 121C based on the cleaning heating profile (step S106: YES), the MCU 1 operates the heating unit 121C based on the cleaning heating profile in response to the removal of the stick-type base material 150 (step S107). On the other hand, if the predetermined period has not elapsed since the previous operation of the heating unit 121C based on the cleaning heating profile (step S106: NO), the MCU 1 does not operate the heating unit 121C and ends this flow.

### Modified Example 1

Based on the SOC of the power supply unit 111C, the MCU 1 may determine whether or not to operate the heating unit 121C based on the cleaning heating profile. In detail, the MCU 1 determines whether the SOC of the power supply unit 111C is equal to or greater than a predetermined value. The MCU 1 operates the heating unit 121C based on the cleaning heating profile in response to a determination that the stick-type base material 150 has been removed from the housing unit 140C after the operation of the heating unit 121C based on the stick heating profile has ended, and that the SOC is equal to or greater than the predetermined value. Here, the specified value of the SOC is, for example, the sum of the power required to execute the current operation of the heating unit 121C based on the cleaning heating profile, and the power required to execute the next operation of the heating unit 121C based on the stick heating profile. If a predetermined value for the SOC is set in this way, the possibility that the SOC of the power supply unit 111C will be reduced by performing heating control based on the cleaning heating profile this time, and that it will not be possible to perform the next heating control based on the stick heating profile will be eliminated.

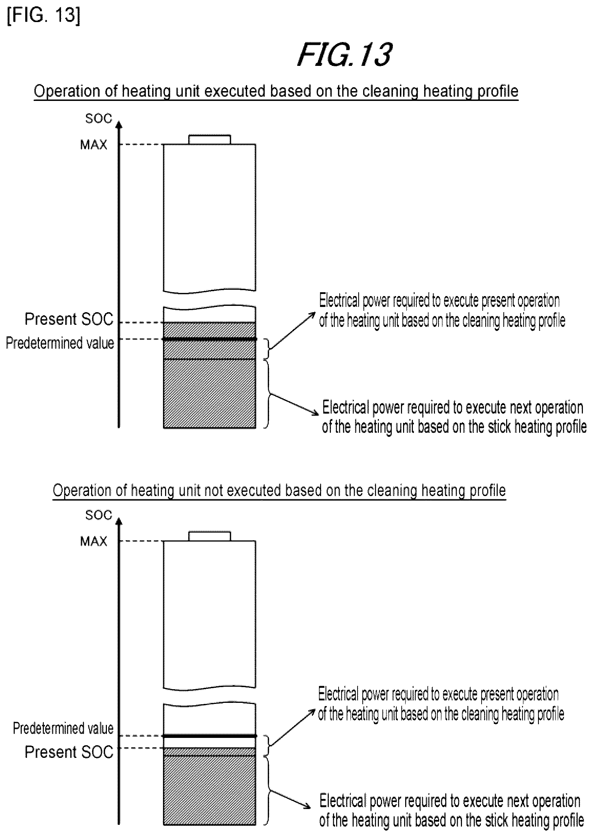

A modified example 1 is described specifically with reference to FIG. 13. As shown in the upper part of FIG. 13, when the SOC at the end of heating control based on the stick heating profile (also referred to as the present SOC) is equal to or greater than the predetermined value described above, it is possible to heat the stick-type base material 150 that is housed next time without charging, even if the operation of the heating unit 121C based on the cleaning heating profile is executed. Therefore, the MCU 1 operates the heating unit 121C based on the cleaning heating profile in response to the determination that the stick-type base material 150 has been removed from the housing unit 140C and that the SOC is equal to or greater than the predetermined value. On the other hand, as shown in the lower part of FIG. 13, when the SOC at the end of heating control based on the stick heating profile (also referred to as the present SOC) is less than the specified value described above, when the operation of the heating unit 121C based on the cleaning heating profile is executed, it will not be possible to heat the stick-type base material 150 that is housed next time due to insufficient power. Therefore, when the MCU 1 determines that the SOC is less than the predetermined value, the MCU 1 does not operate the heating unit 121C based on the cleaning heating profile.

### Modified Example 2

In the embodiment as described above, if the brightness of the reflected light becomes less than a predetermined value L1 after the operation of the heating unit 121C based on the stick heating profile is terminated, the MCU 1 does not detect the stick-shaped substrate 150 and operates the heating unit 121C based on the cleaning heating profile. Modified example 2 differs from the embodiment described above in that in some cases the MCU 1 may not operate the heating unit 121C even if the brightness of the reflected light is less than the predetermined value L1.

When there is a large amount of cleaning agent or dirt inside the housing unit 140C, which occurs as the inhalation device 100 is used, the brightness of the reflected light may be lower than when there is a small amount of cleaning agent or dirt. The reason for this is thought to be because when the light emitted from the stick detection sensor 12 reflects off the inner walls of the housing unit 140C, it is scattered by the cleaning agent and dirt, reducing the amount of light returning to the stick detection sensor 12. In modified example 2, when it is determined based on the brightness of the reflected light that there is a small amount of cleaning agent or dirt in the housing unit 140C, the MCU 1 does not operate the heating unit 121C.

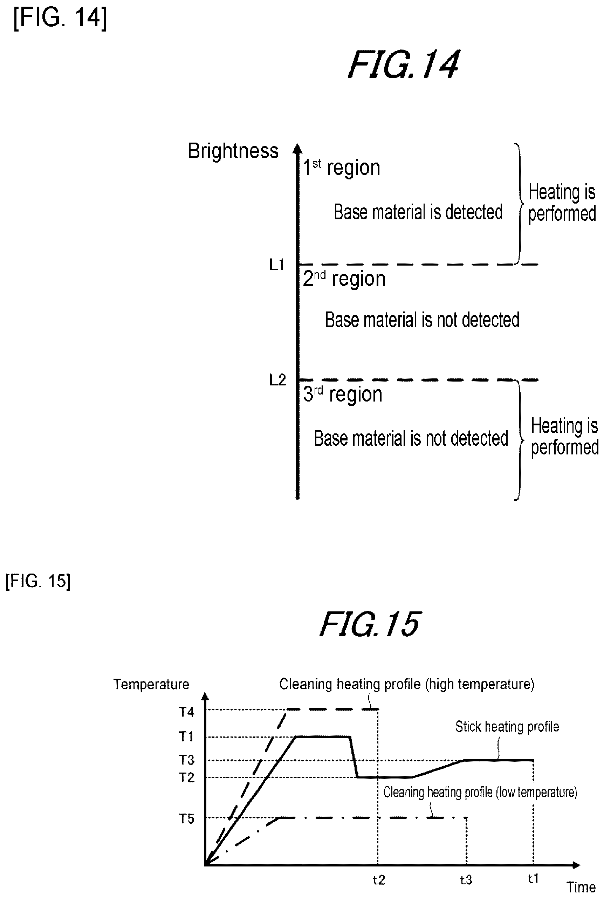

The following is a specific explanation of modified example 2. First, the brightness regions of reflected light detected by the stick detection sensor 12 are explained. As shown in FIG. 14, when the brightness of the reflected light falls within a first range (a range where the brightness is L1 or more), the MCU 1 detects the stick-type base material 150. Also, when the brightness of the reflected light is within a second region (region where the brightness is equal to or greater than L2 and less than L1) or a third region (region where the brightness is less than L2), the MCU 1 does not detect the stick-type base material 150. Here, L2 has a value smaller than L1. These regions are different from each other. The second region has less cleaning agent and dirt in the housing unit 140C and has high brightness of reflected light, whereas in contrast the third region has more cleaning agent and dirt in the housing unit 140C and has low brightness of reflected light.

When the brightness detected by the stick detection sensor 12 is within the first region, the MCU 1 detects the stick-type base material 150, and operates the heating unit 121C based on the stick heating profile as in the embodiment described above.

Also, when the brightness is within the second region, the MCU 1 does not detect the stick-type base material 150, and does not operate the heating unit 121C in response to the stick-type base material 150 being removed from the housing unit 140C. In this way, when it is determined that there is little cleaning agent or dirt in the housing unit 140C, the MCU 1 does not operate the heating unit 121C, thereby reducing power consumption.

Also, when the brightness is within the third region, the MCU 1 does not detect the stick-type base material 150 and operates the heating unit 121C in response to the stick-type base material 150 being removed from the housing unit 140C. In this way, if it is determined that there is a large amount of cleaning agent or dirt in the housing unit 140C, the MCU 1 can operate the heating unit 121C based on the cleaning heating profile to evaporate and remove the liquid substance in the housing unit 140C.

Note that the control method of the inhalation device 100 in the embodiment and modified examples 1 and 2 as described above can be realized by executing a program on a computer (processor) that was prepared in advance. The program is stored in a computer-readable storage medium, and is executed by being read out from the storage medium. The program may also be provided in a form stored in a non-transitory storage medium such as a flash memory, or may be provided over a network such as the Internet. Also, the computer that executes this program may be, for example, included in the inhalation device 100 (for example, the MCU 1), but this is not a limitation, and it may also be included in another device (for example, a smartphone or a server device) that can communicate with the inhalation device 100.

An embodiment of the present invention has been described above with reference to the drawings, but it goes without saying that the present invention is not limited to this embodiment. It is obvious that a person skilled in the art will be able to conceive of a number of variant examples or modified examples within the scope disclosed in the claims, and any such variant examples or modified examples are naturally understood to fall within the technical scope of the present disclosure. Furthermore, the components in the embodiment described above may be arbitrarily combined within a range not deviating from the spirit of the invention.

For example, in the embodiment as described above, the MCU 1 operates the heating unit 121C based on the stick heating profile and the cleaning heating profile, but this is not a limitation. The MCU 1 may operate the heating unit 121C based on information that is not a time series progression (for example, information that does not include time information and specifies only the target temperature of the heating unit 121C), and not a heating profile, which is information that specifies the time series progression of the target temperature.

Also, as shown in the dash-dotted line in FIG. 15, the target temperature of the heating unit 121C in the reference cleaning heating profile may be set lower than the target temperature of the heating unit 121C in the stick heating profile. In this case, the maximum temperature T5 of the cleaning heating profile is set to, for example, about 100°C to 200°C, which is equal to or higher than the boiling point of water, so that the moisture in the housing unit 140C can be evaporated. In this case, although the operation time t3 included in the cleaning heating profile is longer than the operation time t2 in the embodiment as described above, provided the operation time is long enough to evaporate the moisture in the housing unit 140C, t3 can be set shorter than the operation time t1 of the stick heating profile.

Also, the MCU 1 may operate the heating unit 121C based on the cleaning heating profile during the period from when the shutter 23 is opened until the stick detection sensor 12 detects the stick-type base material 150. This enables the moisture in the housing unit 140C to be evaporated before the stick-type base material 150 is heated.

Also, the MCU 1 does not have to automatically operate the heating unit 121C based on the cleaning heating profile in response to the stick-type base material 150 being removed from the housing unit 140C after operation of the heating unit 121C based on the stick heating profile has ended. Specifically, after the stick-type base material 150 is removed from the housing unit 140C, the MCU 1 may operate the heating unit 121C based on a cleaning heating profile in response to a heating request from the user (for example, by pressing the operation unit 24).

Also, in the embodiment as described above, an optical sensor is described as an example of the stick detection sensor 12, but this is not a limitation. For example, the stick detection sensor 12 may be a pressure sensor that detects changes in pressure within the housing unit 140C associated with the insertion and removal of the stick-type base material 150. In this case, the MCU 1 detects the stick-type base material 150 based on fluctuations in pressure detected by the pressure sensor. Also, in the case where identification information is attached to the stick-type base material 150, the stick detection sensor 12 may be an identification information reader capable of reading the identification information on the stick-type base material 150. In this case, the MCU 1 detects the stick-type base material 150 based on the results of the reading by the identification information reader. Also, the stick detection sensor 12 may be a mechanical switch that is provided near the housing unit 140C (for example, on the bottom surface of the housing unit 140C) and is depressed by the stick-type base material 150. In this case, the MCU 1 detects the stick-type base material 150 by the switch being depressed. Also, if the stick-type base material 150 contains a susceptor, the MCU 1 may detect the stick-type base material 150 based on a change in the characteristics of the circuit of the inhalation device 100 (for example, a change in inductance) due to the insertion of the stick-type base material 150.

The present specification and so on sets forth at least the following features. Corresponding components and so on in the embodiment described above are shown by way of example in parentheses, but are not limited thereto.
(1) An inhalation device (inhalation device 100, 100A, 100B) for generating an aerosol from a base material (stick-type base material 150) having an aerosol source, the inhalation device (inhalation device 100, 100A, 100B) comprising:
   a housing unit (housing unit 140, 140C) that houses the base material;
   a heating unit (heating unit 121A to 121C) that heats the housing unit; and
   a control unit (control unit 116A, 116B, MCU 1) that controls the heating unit,
   wherein the control unit
      operates the heating unit when the base material is housed in the housing unit, and
      operates the heating unit in response to the base material being removed from the housing unit after completion of an operation of heating the base material.
   Dirt that occurs in the housing unit as the inhalation device is used includes parts of the aerosol source that have fallen off the base material and have adhered to the housing unit together with liquid, and part of the aerosol generated by heating the base member that becomes liquid and adheres to the housing unit. According to (1), the control unit operates the heating unit in response to the base material being removed from the housing unit after completion of an operation of heating of the base material. In this way, the liquid in the housing unit is evaporated and removed, so it is possible to remove the dirt adhering to the inside of the housing unit, or it becomes easier to remove the dirt. Therefore, the convenience of cleaning the inhalation unit can be improved.
(2) The inhalation device according to (1), wherein
   the control unit controls the heating unit based on heating information that prescribes the time series transition of a target temperature that is a target value of the temperature of the heating unit,
   the heating information includes at least first heating information (stick heating profile) for heating the base material, and second heating information (cleaning heating profile) that is different from the first heating information,
   and the control unit
      operates the heating unit based on the first heating information when the base material is housed in the housing unit, and
      operates the heating unit based on the second heating information in response to removal of the base material from the housing unit after completion of an operation of the heating unit based on the first heating information.
   According to (2), the heating unit is operated based on appropriate heating information in accordance with the circumstances, so more appropriate heating control can be executed.
(3) The inhalation device according to (2), wherein
   the first heating information and the second heating information each include operation time for operating the heating unit, and
   the operation time of the second heating information (operation time t2) is shorter than the operation time of the first heating information (operation time t1).
   According to (3), when the heating unit is operated based on the second heating information, excessive heating of the heating unit from which the base material has been removed is reduced.
(4) The inhalation device according to (3), wherein
   the target temperature of the second heating information is higher than the target temperature of the first heating information.
   According to (4), the target temperature of the second heating information is higher than the target temperature of the first heating information, so the operation time of the second heating information can be shortened.
(5) The inhalation device according to any one of (1) to (4), wherein
   after a predetermined period of time has elapsed from the previous operation of the heating unit that was executed in response to the base material being removed from the housing unit, the control unit operates the heating unit in response to the base material being removed from the housing unit after completion of the operation of heating the base material.
   According to (5), heating control is not performed continuously, so electrical power consumption can be reduced compared with executing heating control every time the base material is removed.
(6) The inhalation device according to any one of (1) to (5), further comprising
   a power supply unit (power supply unit 111A to 111C) that can supply electrical power to the heating unit, wherein
   the control unit
   determines whether or not the remaining capacity of the power supply unit is equal to or greater than a predetermined value, and
   operates the heating unit in response to the base material being removed from the housing unit after completion of the operation of heating of the base material, and determining that the remaining capacity is equal to or greater than the predetermined value.
   According to (6), it is possible to determine whether or not to operate the heating unit in response to the base material being removed from the housing unit taking into consideration the remaining capacity of the power supply unit.
(7) The inhalation device according to any one of (1) to (6), wherein
   the control unit is capable of selectively switching between a first mode in which the heating unit is operated in response to the base material being removed from the housing unit after completion of the operation of heating the base material, and a second mode in which the heating unit is not operated even if the base material is removed from the housing unit after completion of the operation of heating the base material.
   According to (7), the control unit selectively takes the first mode or the second mode, so it is possible to reflect a user's wish to not control the heating after the base material is removed.
(8) The inhalation device according to any one of (1) to (7), further comprising
   an optical sensor (stick detection sensor 12) for emitting light to the housing unit and detecting the amount of light reflected from the housing unit, wherein
   the control unit is configured to be capable of detecting the base material housed in the housing unit based on the amount of reflected light.
   According to (8), the base material housed in the housing unit can be detected based on the amount of reflected light detected by the optical sensor.
(9) The inhalation device according to (8), wherein
   the control unit
   detects the base material and operates the heating unit when the amount of light is within a first region,
   does not detect the base material and does not operate the heating unit in response to the base material being removed from the housing unit when the amount of light is in a second region that is different from the first region, and
   does not detect the base material and operates the heating unit in response to the base material being removed from the housing unit when the amount of light is in a third region that is different from the first region and the second region.
   According to (9), when the base material is removed from the housing unit, the heating unit is not operated when heating is not necessary, so power consumption can be reduced.
(10) The information processing device according to (8) or (9), further comprising
   a flexible member (sensor FPC 73) electrically connected to the control unit and arranged around the housing unit, wherein
   the optical sensor is provided on the flexible member.
   According to (10), by providing the optical sensor on the flexible member, the degree of freedom of arranging the optical sensor around the housing unit is increased compared with, for example, providing the optical sensor on a rigid board.
(11) The inhalation device according to (10), wherein
   a transparent member (transmission filter 311) that can transmit light is provided on a portion of the wall that delimits the housing unit, and
   the flexible member is arranged around the housing unit so that the optical sensor faces the transparent member at a predetermined distance.
   According to (11), the optical sensor faces the transparent member at the predetermined distance, so it is possible to reduce the effect of heat from the housing unit on the optical sensor.
(12) The inhalation device according to any one of (1) to (11), further comprising
   a notification unit (light emitting unit 25, vibration device 60) for notifying the user that the heating unit is operating, wherein
   the notification unit notifies the user that the heating unit is operating when the heating unit is operating after the base material has been removed from the housing unit.
   According to (12), when the heating unit is operating after the base material has been removed from the housing unit, the user can easily determine that heating control is being performed, and, for example, can take care not to put their fingers close to the housing unit.
(13) The inhalation device according to (12), wherein
   the notification unit
   notifies the user by a first notification mode that the heating unit is operating when the heating unit is operating while the base material is housed in the housing unit, and
   notifies the user by a second notification mode that is different from the first notification mode that the heating unit is operating when the heating unit is operating based on the second heating information after the base material has been removed from the housing unit.
   According to (13), the user can easily determine that the heating unit is operating while the base material is not housed in the housing unit, so, for example, the user can take care not to bring their fingers close to the housing unit
(14) A control method executed by a computer (control unit 116A, 116B, MCU 1) that controls operation of an inhalation device (inhalation device 100, 100A, 100B) that generates an aerosol from a base material (stick-type base material 150) having an aerosol source, wherein
   the inhalation device comprises:
      a housing unit (housing unit 140, 140C) that houses the base material; and
      a heating unit (heating unit 121A to 121C) that heats the housing unit, and
   the computer
      operates the heating unit when the base material is housed in the housing unit, and
      operates the heating unit in response to the base material being removed from the housing unit after completion of the operation of heating the base material.
   As the inhalation device is used, dirt may accumulate in the housing unit, such as parts of the aerosol source that have fallen off the base material and adhere to the housing unit together with the liquid, or part of the aerosol generated by heating the base material may become liquid and adhere to the housing unit. According to (14), the computer operates the heating unit in response to the base material being removed from the housing unit after completion of operation of the heating unit heating the base material. In this way, the liquid in the housing unit is evaporated and removed, so it is possible to remove the dirt adhering to the inside of the housing unit, or it becomes easier to remove the dirt. Therefore, the convenience of cleaning the inhalation unit can be improved.
(15) A program that causes a computer (control unit 116A, 116B, MCU 1) that controls operation of an inhalation device (inhalation device 100, 100A, 100B) that generates aerosol from a base material (stick-shaped base material 150) having an aerosol source to perform a predetermined process, wherein
   the inhalation device comprises:
      a housing unit (housing unit 140, 140C) that houses the base material; and
      a heating unit (heating unit 121A to 121C) that heats the housing unit, and
   the computer is caused to perform the process of
      operating the heating unit when the base material is housed in the housing unit, and
      operating the heating unit in response to the base material being removed from the housing unit after completion of the operation of heating the base material.
   As the inhalation device is used, dirt may accumulate in the housing unit, such as parts of the aerosol source that have fallen off the base material and adhere to the housing unit together with the liquid, or part of the aerosol generated by heating the base material may become liquid and adhere to the housing unit. According to (15), the computer operates the heating unit in response to the base material being removed from the housing unit after completion of operation of the heating unit heating the base material. In this way, the liquid in the housing unit is evaporated and removed, so it is possible to remove the dirt adhering to the inside of the housing unit, or it becomes easier to remove the dirt. Therefore, the convenience of cleaning the inhalation unit can be improved.
(16) An inhalation system, comprising: a base material (stick-type base material 150) having an aerosol source; and
   an inhalation device (inhalation device 100, 100A, 100B) according to any one of (1) to (13).
   As the inhalation device is used, dirt may accumulate in the housing unit, such as parts of the aerosol source that have fallen off the base material and adhere to the housing unit together with the liquid, or part of the aerosol generated by heating the base material may become liquid and adhere to the housing unit. According to (16), the control unit operates the heating unit in response to the base material being removed from the housing unit after completion of operation of the heating unit to heat the base material. In this way, the liquid in the housing unit is evaporated and removed, so it is possible to remove the dirt adhering to the inside of the housing unit, or it becomes easier to remove the dirt. Therefore, the convenience of cleaning the inhalation unit can be improved.
(17) The inhalation device according to (13), wherein
   the notification unit comprises a light emitting unit (light emitting unit 25) that notifies users by emitting light, and
   the first notification mode and the second notification mode are different light emission patterns.
   According to (17), the user can visually and easily determine whether heating control to heat the base material is being performed, or whether heating control is being performed after the base material has been removed from the housing unit.
(18) The inhalation device according to (17), wherein
   the first notification mode and the second notification mode are different light emission colors of the light emitting unit.
   According to (18), the user can easily determine whether heating control is being performed to heat the base material, or whether heating control is being performed after the base material has been removed from the housing unit by checking the color of the emitted light.
(19) The inhalation device according to (17), wherein
   the light emitting unit has a plurality of light emitting elements (LED 251), and
   the first notification mode and the second notification mode have different numbers of light emitting elements that emit light.
   According to (19), the user can easily determine whether heating control is being performed to heat the base material or heating control is being performed after the base material has been removed from the housing unit by checking the number of light-emitting elements that are emitting light.

### REFERENCE SIGNS LIST

1 MCU (control unit, computer)
12 Stick detection sensor (optical sensor)
25 Light emitting unit (notification unit)
60 Vibration device (notification unit)
73 Sensor FPC (flexible member)
100, 100A, 100B Inhalation device
116A, 116B Control unit
121A to 121C Heating unit
140, 140C Housing unit
150 Stick-type base material (base material)
311 Transmission filter (transparent member)

## Claims

1. An inhalation device for generating an aerosol from a base material having an aerosol source, the inhalation device comprising:
a housing unit that houses the base material;
a heating unit that heats the housing unit; and
a control unit that controls the heating unit,
wherein the control unit
operates the heating unit when the base material is housed in the housing unit, and
operates the heating unit in response to the base material being removed from the housing unit after completion of an operation of heating the base material.

2. The information processing device according to claim 1, wherein
the control unit controls the heating unit based on heating information that prescribes the time series transition of a target temperature that is a target value of the temperature of the heating unit,
the heating information includes at least first heating information for generating an aerosol from the base material, and second heating information that is different from the first heating information,
the control unit
operates the heating unit based on the first heating information when the base material is housed in the housing unit, and
operates the heating unit based on the second heating information in response to removal of the base material from the housing unit after completion of operation of the heating unit based on the first heating information.

3. The inhalation device according to claim 2, wherein
the first heating information and the second heating information each include operation time for operating the heating unit, and
the operation time of the second heating information is shorter than the operation time of the first heating information.

4. The inhalation device according to claim 3, wherein
the target temperature of the second heating information is higher than the target temperature of the first heating information.

5. The inhalation device according to any one of claims 1 to 4, wherein
after a predetermined period of time has elapsed from the previous operation of the heating unit that was executed in response to the base material being removed from the housing unit, the control unit operates the heating unit in response to the base material being removed from the housing unit after completion of the operation of heating the base material.

6. The inhalation device according to any one of claims 1 to 5, further comprising
a power supply unit that can supply electrical power to the heating unit, wherein
the control unit
determines whether or not the remaining capacity of the power supply unit is equal to or greater than a predetermined value, and
operates the heating unit in response to the base material being removed from the housing unit after completion of the operation of heating of the base material, and determining that the remaining capacity is equal to or greater than the predetermined value.

7. The inhalation device according to any one of claims 1 to 6, wherein
the control unit is capable of selectively switching between a first mode in which the heating unit is operated in response to the base material being removed from the housing unit after completion of the operation of heating the base material, and a second mode in which the heating unit is not operated even if the base material is removed from the housing unit after completion of the operation of heating the base material.

8. The inhalation device according to any one of claims 1 to 7, further comprising
an optical sensor for emitting light to the housing unit and detecting the amount of light reflected from the housing unit, wherein
the control unit is configured to be capable of detecting the base material housed in the housing unit based on the amount of reflected light.

9. The inhalation device according to claim 8, wherein
the control unit
detects the base material and operates the heating unit when the amount of light is within a first region,
does not detect the base material and does not operate the heating unit in response to the base material being removed from the housing unit when the amount of light is in a second region that is different from the first region, and
does not detect the base material and operates the heating unit in response to the base material being removed from the housing unit when the amount of light is in a third region that is different from the first region and the second region.

10. The inhalation device according to claim 8 or 9, further comprising
a flexible member electrically connected to the control unit and arranged around the housing unit, wherein
the optical sensor is provided on the flexible member.

11. The inhalation device according to claim 10, wherein
a transparent member that can transmit light is provided on a portion of the wall that delimits the housing unit, and
the flexible member is arranged around the housing unit so that the optical sensor faces the transparent member at a predetermined distance.

12. The inhalation device according to any one of claims 1 to 11, further comprising
a notification unit for notifying the user that the heating unit is operating, wherein
the notification unit notifies the user that the heating unit is operating when the heating unit is operating after the base material has been removed from the housing unit.

13. The inhalation device according to claim 12, wherein
the notification unit
notifies the user by a first notification mode that the heating unit is operating when the heating unit is operating while the base material is housed in the housing unit, and
notifies the user by a second notification mode that is different from the first notification mode that the heating unit is operating when the heating unit is operating after the base material has been removed from the housing unit.

14. A control method executed by a computer that controls operation of an inhalation device that generates an aerosol from a base material having an aerosol source, wherein
the inhalation device comprises:
a housing unit that houses the base material;
a heating unit that heats the housing unit, and
the computer
operates the heating unit when the base material is housed in the housing unit, and
operates the heating unit in response to the base material being removed from the housing unit after completion of the operation of heating the base material.

15. A program that causes a computer that controls operation of an inhalation device that generates aerosol from a base material having an aerosol source to perform a predetermined process, wherein
the inhalation device comprises:
a housing unit that houses the base material;
a heating unit that heats the housing unit, and
the computer is caused to perform the process of
operating the heating unit when the base material is housed in the housing unit, and
operating the heating unit in response to the base material being removed from the housing unit after completion of the operation of heating the base material.
